# EUROPEAN PATENT APPLICATION

(11) **EP 4 382 148 A1**
(43) Date of publication of application: **12.06.2024**
(21) Application number: 23213281.1
(22) Date of filing: 30.11.2023
(51) Int. Cl.: A61M 5/142, A61M 5/145, A61M 5/20, A61M 5/315, A61M 5/31

(54) **LEAD SCREW WITHIN RESERVOIR**

(30) Priority: 06.12.2022 US 202263386249 P
(71) Applicant: Insulet Corporation, Acton MA 01720 (US)
(72) Inventor: BROWN, James, Temecula, CA (US)
(74) Representative: Peterreins Schley

(57) **Abstract**

Disclosed are techniques, devices and systems that provide a compact reservoir and plunger arrangement. In the disclosed examples, a reservoir may include a housing, a plunger, a lead screw and an elastic sleeve. The housing may have a first end and a second end. The plunger may be configured to fit within the housing. The leadscrew may be coupled to the plunger. The elastic sleeve may surround the leadscrew and be coupled to a surface of the plunger and to a surface of the second end of the housing. The elastic sleeve is leakproof.

## Description

### BACKGROUND

For wearable medical devices, physical size is a major driver of the overall impact of therapy on the patients employing them. One of the largest elements in a complex and space-constrained medical device (such as an insulin pump) is the reservoir and plunger arrangement. To maintain a small device footprint, a design objective is to optimize the volume occupied by the reservoir and plunger of a drug delivery device.

Using a screw to drive a plunger is advantageous for accurate dispensing from a reservoir. When the screw is in line with the plunger, it effectively doubles the length of the plunger making the packaging/overall size larger. Putting the screw inside of the reservoir is challenging because sealing on the threads of the plunger in addition to the O-ring on the plunger side wall is challenging both technically and for manufacturing. It would be helpful to have a small, low-cost, micro-actuator with reciprocal motion, maximum output force output for a given electrical input.

### BRIEF SUMMARY

The present invention is directed to a drug delivery system as defined in the enclosed claims. This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

According to an example of the disclosed subject matter, a reservoir for a drug delivery system is disclosed. The reservoir may include a cylindrical housing, a plunger, an elastic sleeve, a lead screw and a reservoir cap. The plunger may be configured to fit within the cylindrical housing. The elastic sleeve may be within the cylindrical housing and be coupled to a surface of the plunger. The elastic sleeve is leakproof. The lead screw may pass through a center of the elastic sleeve and a center of the plunger. The reservoir cap may be coupled to the cylindrical housing.

According to an example of the disclosed subject matter, another reservoir for a drug delivery system is disclosed. The reservoir may include a rigid housing, a plunger, a lead screw and an elastic sleeve. The rigid housing may have a first end and a second end. The plunger may be configured to fit within the rigid housing. The leadscrew may be coupled to the plunger. The elastic sleeve may surround the leadscrew and be coupled to a surface of the plunger and to a surface of the second end of the rigid housing. The elastic sleeve is leakproof.

### BRIEF DESCRIPTION OF THE DRAWINGS

FIG. 1A illustrates an example of a reservoir for a drug delivery system according to the disclosed subject matter.
FIG. 1B illustrates an exemplary initial or prefill position and final position or expelled position of the reservoir shown in FIG. 1A.
FIG. 1C illustrates an exploded view of the example of the reservoir of FIG. 1A.
FIG. 1D illustrates an exploded view of the example of the reservoir of FIG. 1A.
FIG. 1F illustrates an exploded view of the example of the reservoir of FIG. 1A.
FIG. 2A illustrates a detailed view of an arrangement of a plunger and base washer usable in the examples of a reservoir shown in earlier embodiments.
FIG. 2B illustrates a general view of the example of a reservoir of FIG. 2A.
FIG. 3 illustrates a number of features related to an exemplary locking feature according to the disclosed subject matter.
FIG. 4A illustrates an exemplary locking feature for a drug delivery device reservoir according to the disclosed subject matter.
FIG. 4B illustrates further details of the exemplary locking feature illustrated in FIG. 4A.
FIG. 4C illustrates further details of the exemplary locking feature illustrated in FIGs. 4A and 4B.
FIG. 5 shows a block diagram of an exemplary wearable drug delivery device suitable for using the reservoir illustrated and described with reference to FIGs. 1A-4C.

### DETAILED DESCRIPTION

The following discussion provides a detailed discussion of a drug delivery device reservoir. The reservoir examples described herein may include various features related to an elastic sleeve used to enable a lead screw to pass through an interior of the reservoir.

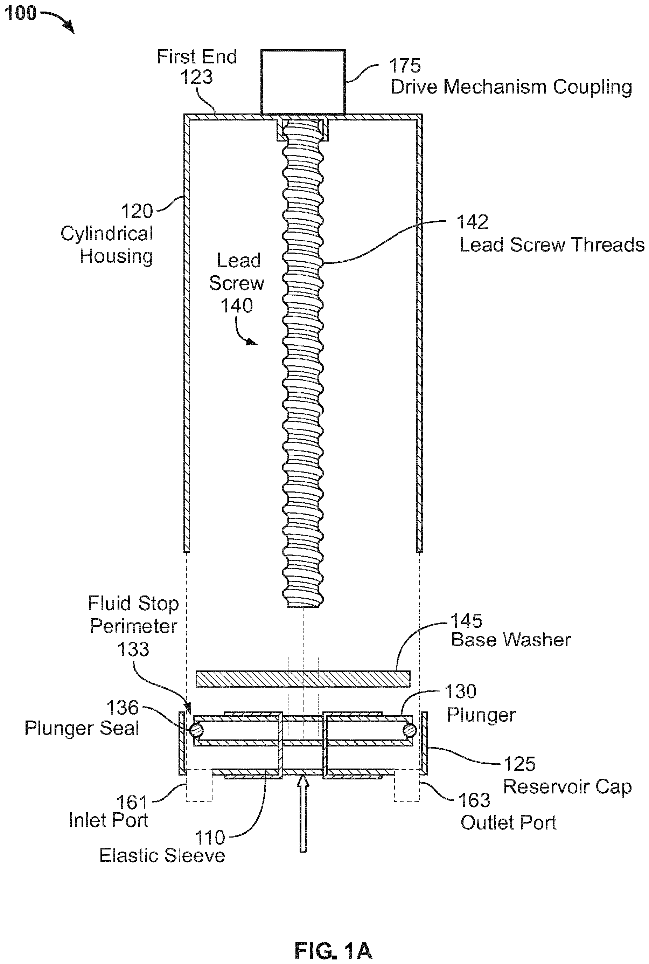

FIG. 1A illustrates an example of a reservoir for a drug delivery system. In FIG. 1A, the reservoir 100 for a drug delivery system may include an elastic sleeve 110, a housing 120, a plunger 130, and a lead screw 140.

The housing 120 may be a rigid housing or structure with a first end and a second end. For example, the housing 120 may be in the shape of a cylinder (i.e., a cylindrical housing) with a first end 123 and a reservoir cap 125, where the reservoir cap 125 is the second end. The cylindrical shape of the housing 120 is not limited to a circular cylinder, but may be oval, rectangular, hexagonal or any other tubular shape. The first end 123 of the housing 120 is opposite the reservoir cap 125 (or second end). The reservoir cap 125 may be coupled to the housing 120 or may be integral to the housing 120. For example, the reservoir cap 125 may be coupled to an end of the housing 120 by bonding, welding, friction, snaps (e.g., snap fittings), threads or the like. The reservoir cap 125 is stationary with respect to the housing 120.

The plunger 130 is configured to fit within the housing 120. The plunger 130 includes a fluid stop perimeter 133. The fluid stop perimeter 133 may have a plunger seal 136. For example, the fluid stop perimeter 133 may be a detent or the like in the perimeter of the plunger 130. The shape of fluid stop perimeter 133 may be a semi-circular channel, an arched channel, a square channel or a channel of any shape. The fluid stop perimeter 133 may be filled or occupied by, in place about the plunger 130. The plunger seal 136 may be a leakproof sealing component that contacts the interior of the housing 120. For example, the plunger seal 136 may be an O-ring or similar sealing device. In addition to occupying the fluid stop perimeter 133, the plunger seal 136, which is an example of the leakproof sealing component, is configured to engage an internal wall of the housing 120. In order to effectively function as a leakproof sealing component, the plunger seal 136 is configured to engage the entire internal wall of the housing 120.

The elastic sleeve 110 may be configured as a tube with two open ends. For example, the elastic sleeve 110 may be positioned within the housing 120 and have a first tube end coupled to the reservoir cap 136 and a second tube end, opposite the first tube end, coupled to a surface of the plunger 130. The elastic sleeve 110 is made from a leakproof material, such as, for example, a silicone rubber, flexible polyvinyl chloride (PVC) tubing, or a similar elastic or flexible material and may have an approximate wall thickness of the elastic sleeve 110 may be approximately 1/2 to 1/4 millimeters or the like. The material and the wall thickness of the elastic sleeve 110 may be selected so that the elastic sleeve 110 may fold over on itself as the drug is pushed out. As a result, there is very little hold-up volume of the liquid drug due to the thin walls of elastic sleeve 110. Hold-up volume may be considered an amount of liquid drug that remains after the plunger 130 is no longer able to expel the liquid drug from the reservoir 110.

The lead screw 140 is configured to pass through a center of the elastic sleeve 110 and a center of the plunger 130. The lead screw 140 may have threads, such as lead screw threads 142. The number of threads 142 enables precision movement of the plunger 130 and precise control of an amount of liquid drug that is expelled from the reservoir 100. The lead screw 140 is operable to move the plunger within the housing 120 via engagement of the lead screw 140 with a drive mechanism coupling 175. The drive mechanism coupling 175 may be a link or connection that is operable to transfer forces generated by a drive mechanism to the lead screw 140 in response to which lead screw 140 rotates.

In the illustrated example, the lead screw 140 may also be coupled to a base washer 145. In an example, the base washer 145 is configured with a hole so the lead screw 140 may pass through. The hole in the base washer 145 may be threaded to match the lead screw threads 142. When threaded, the base washer 145 is operable to apply pressure to the plunger 130 or relieve pressure depending upon the direction of rotation of the lead screw 140.

The liquid drug may flow into and out of the reservoir 100. FIG. 1A also shows examples of ports that enable the liquid drug to be input to the reservoir 100, such as inlet port 161, and to be expelled from the reservoir 100, such as outlet port 163. Other than being able to enable the liquid drug to pass into the reservoir 100 and permit the liquid drug to pass from the reservoir 100, the details of inlet port 161 and outlet port 163 are not necessary for understanding the claimed subject matter.

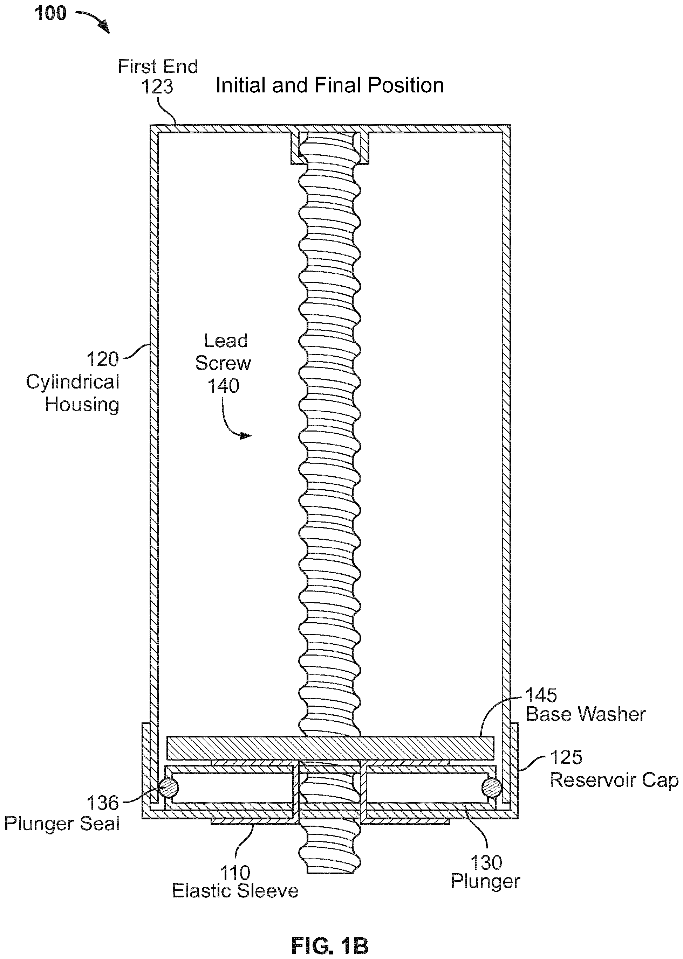

FIG. 1B illustrates an exemplary initial or prefill position and final position or expelled position of the reservoir shown in FIG. 1A. As shown in FIG. 1B, the reservoir 100 has an initial position when the plunger 130 is located at a location near to, or within, the reservoir cap 125 within the housing 120. The base washer 145 may be positioned farthest away from the first end 123 of the housing 120. The elastic sleeve 110 is shown in a contracted state. A "top portion" of the elastic sleeve 110 is shown coupled between the base washer 145 and the plunger 130, while a "bottom portion" of the elastic sleeve 110 is shown coupled to an exterior of the reservoir cap 125. The coupling of the elastic sleeve 110 between the base washer 145 and the plunger 130 may be accomplished by affixing an end portion of the elastic sleeve 110 to a surface of the plunger 130 or to a surface of the base washer 145. The lead screw 140 may pass through the elastic sleeve 110. A lubricant, such as silicone grease, graphite or the like, may be applied to the lead screw 140 to eliminate the potential for the elastic sleeve 110 to catch or otherwise bind on a thread or other part of the lead screw 140. In some examples, the lead screw 140 may extend through the reservoir cap 125 and beyond the length of the housing 120.

While the base washer 145 and the plunger 130 are called out separately, the two elements may be formed as a single component. When the base washer 145 and the plunger 130 are formed as a single component (i.e., base washer/plunger), the elastic sleeve 110 may extend to a top portion of the base washer/plunger (closest to the first end 123).

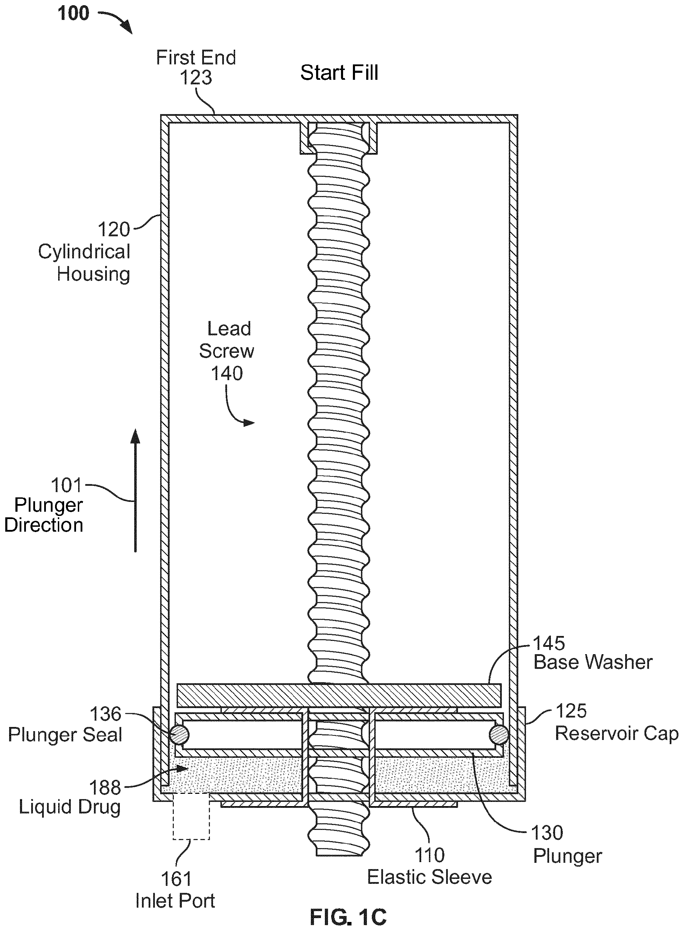

FIG. 1C illustrates a view of the exemplary reservoir of FIGs. 1A and 1B when it is starting to fill.

When the reservoir 100 is starting to be filled, the elastic sleeve 110 is operable to expand as the plunger 130 is moved away in the plunger direction 101 (as well as movement of the base washer 145 in the same direction as the plunger direction 101) from the reservoir cap 136. The movement of the plunger 130 in direction 101 is in response to rotation of the lead screw 140 in a direction away from the reservoir cap 125. In one or more examples, the reservoir 100 may be filled with a liquid drug 188 via the inlet port 161.

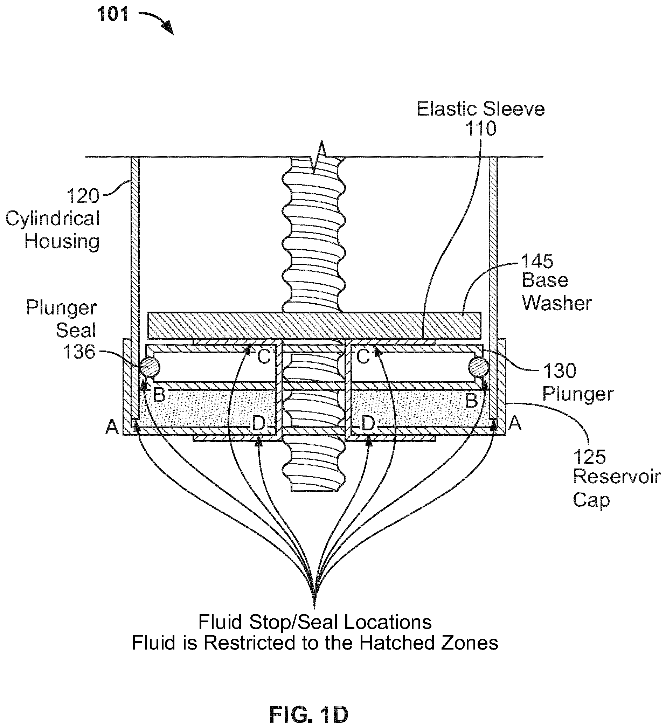

FIG. 1D illustrates a detailed view of examples of fluid seal locations in the exemplary reservoir of FIGs. 1A-C.

The detailed view 101 shows the elastic sleeve 110, a portion of the housing 120, the reservoir cap 125, the plunger 130, the liquid drug 188, and different fluid stop/seal locations A-E. As shown in more detail in FIG. 1D, the elastic sleeve 110 is positioned between the base washer 145 and the plunger 130, and extends radially through a center opening (shown in later examples) of the plunger 130. In this example, a first part of the elastic sleeve 110 may be sealed at a surface of the plunger 130 facing the base washer 145 and a second part of the elastic sleeve 110 may be sealed at a surface of the reservoir cap 125. The liquid drug 188 is shown in a space made by the expansion of the elastic sleeve 110.

One or more areas for potential leakage in the reservoir 100 may be sealed by different methods at the respective fluid stop/seal locations A-E. Note that the elastic sleeve 110, housing 120, plunger 130 and plunger seal 136, and the reservoir cap 125 are all cylindrical so the left and right sides of FIG. 1D may be a single point anywhere along a circumference of the respective component. For example, there may be an intersection between the reservoir cap 125 and the cylindrical housing at which the reservoir cap 125 may be welded, secured by threads, or adhered to the housing 120 at fluid stop/seal location A. Any potential for leaks around the plunger 130 may be prevented by plunger seal 136 at fluid stop/seal location B which fills the gap between the entire perimeter of the plunger 130 and the internal circumference of the housing 120. Fluid stop/seal locations C and D also extend around the circumference of the respective first part and second part of the elastic sleeve 110. Any liquid drug 188 in the reservoir 100 is limited to the cross hatched areas where the liquid drug 188 is shown. The base washer 145 may be threaded and function as a "nut" on the lead screw 140. The base washer 145 may be configured to be secured to the plunger 130 via threads on part of the plunger, by adhesive, ultrasonic welding, laser welding, or the like.

FIG. 1F illustrates a partially filled view of the exemplary reservoir of FIGs. 1A-1C.

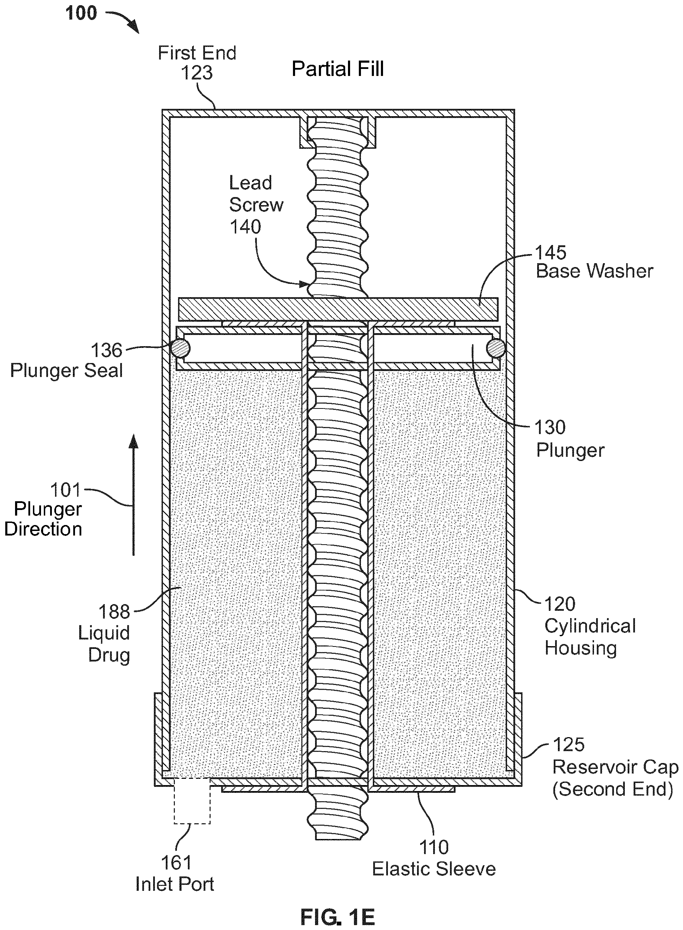

In the example of FIG. 1E, the reservoir 100 is shown in partially-filled with the plunger 130 positioned within the housing 120 away from the reservoir cap 136. When the reservoir 100 is filled, the plunger 130 and base washer 145 are located at a location away from the reservoir cap 136 within the housing 120. To arrive at the state shown in FIG. 1E, the plunger 130 may traverse along the lead screw 140 away from the reservoir cap 125 (or second end of the rigid housing) causing the elastic sleeve 110 to expand, which creates a void to be filled via the inlet port 161 by the liquid drug 188. Conversely, when the liquid drug is expelled from the reservoir 100, the plunger 130 traverses in an opposite direction and the elastic sleeve 110 contracts all the while maintaining a leakproof boundary for the lead screw 140. The inlet port 161 may be configured to pass through the second end 125 of the rigid housing 120 and enable a liquid drug to fill a void between the plunger 130 and the second end 125 when the plunger 130 is moving toward the first end 123.

The elastic sleeve 110 may have an exterior portion that contacts the liquid drug 188 and an interior portion through which the lead screw 140 may pass. The elastic sleeve 110 maintains a boundary between the liquid drug 188 and the leadscrew 140 in the reservoir 100 since the elastic sleeve 110 is leakproof so too is the boundary. The lead screw 140 may have a threaded zone along the length (i.e., from the first end 123 of the housing 120 to the reservoir cap 125) of the lead screw 140 and the threaded zone is outside of any fluid zone (i.e., the cross-hatched areas where the liquid drug 188 is located). When the liquid drug is to be expelled from the reservoir 100, the elastic sleeve 110 is operable to contract as the base washer 145 and the plunger 130 are moved toward the reservoir cap 136. Since the lead screw 140 passes through the elastic sleeve 110, the lead screw 140 may contact the interior portion of the elastic sleeve 110. The interior portion (i.e., the portion of the elastic sleeve 110 closest to the lead screw 140) of the elastic sleeve 110 may have a lubricant, such as silicone grease or the like. In an example, the elastic sleeve 100 surrounds the leadscrew 140 and may be coupled to a surface (e.g., a top surface closest to the base washer 145) of the plunger 130 and to a surface of the second end (e.g., the reservoir cap 125) of the rigid housing 120.

In the examples 1A-1E, the elastic sleeve 110 may be configured in such a manner that a force is needed to cause the elastic sleeve 100 to expand. The force may be applied by the liquid drug 188 flowing into the reservoir 100, by the plunger 130 and base washer 145 pulling the elastic sleeve 100 away from the reservoir cap 125 in response to a rotation of the lead screw 140, or by a combination of both the liquid drug flowing into the reservoir 100 and the pulling by the plunger 130 and base washer 145. Conversely, because the elastic sleeve 110 was elastic, little or no force is required to be applied by an external source to contract the elastic sleeve 110 back. Only the threads on the threaded base washer 145 described above enable the elastic sleeve 110 to maintain its expanded state. Without the threaded base washer 145 being there, the elastic sleeve 110 would apply pressure to liquid drug 188 and may cause the liquid drug 188 to leak from the reservoir 100.

While threading the base washer and/or the plunger may provide a solution to prevent drainage after filling, other mechanisms and/or techniques are envisioned to lock the base washer 145 and/or plunger in place and prevent the elastic sleeve 100 from contracting, which prevents leakage from the reservoir.

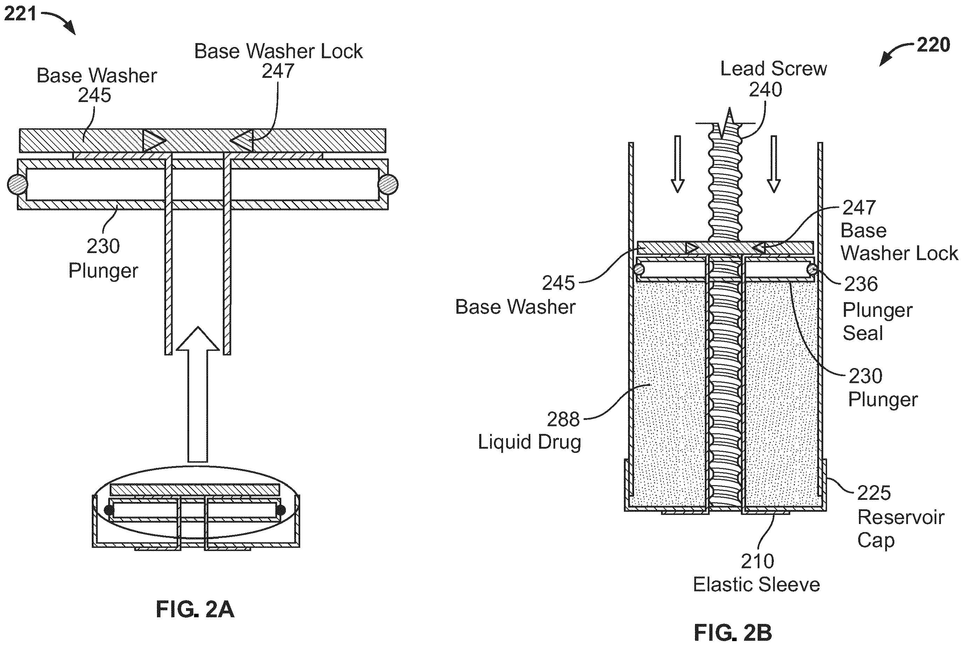

FIG. 2A illustrates a detailed view of an arrangement of a plunger and base washer usable in the examples of a reservoir shown in earlier embodiments.

FIG. 2A shows a locking feature usable in the arrangement 221 of the plunger 230 and base washer 245. In this example, instead of the base washer being threaded, a locking feature is a base washer lock 247 that is configured to lock (in one direction in some examples) the base washer 245 against the lead screw 240. The base washer lock 247 may be a keying device, a square screw fitted into a squared inset of the base washer 245, or the like. A purpose of the locking feature is to prevent the base washer 245 from applying pressure to the plunger 230 without positive rotational movement by the lead screw 240 or in response to vibrations or movement of the user. As a result, inadvertent expulsion of the liquid drug from the reservoir may be avoided. The base washer 245 may be molded or 3-D printed with the base washer lock 247 in place so as to avoid having to assemble the base washer 245 and the base washer lock 247.

FIG. 2B illustrates a general view of the example of a reservoir of FIG. 2A.

The plunger seals 236 and the elastic sleeve 210 seal a portion of the reservoir 220 between the plunger 230 and the reservoir cap 225. The reservoir 220 is shown with an open top that allows the base washer lock 247, which may be a threaded nut, to be accessed (as shown by the pair of arrows). The open top of the reservoir 220 allows for several options to lock or otherwise engage the base washer lock 247 once filling of the reservoir 220 with the liquid drug 288 is completed. The base washer lock 247 performs two functions: 1) it permits the plunger 230 and base washer 245 to rise freely in the direction away from the reservoir cap 225 along the length of the lead screw 240 when the reservoir 220 is being filled with a liquid drug 288, and 2) enables more precise control of the amount of liquid drug 288 being dispensed by locking to the lead screw 240 due to the lead screw 240 having to drive the base washer 247 downward toward the reservoir cap 225.

In an example of when the base washer 247 is a threaded nut, the threads may engage threads of the lead screw 240. The lead screw 240 may be operable to move the threaded nut enabling the elastic sleeve 210 to expand and be filled with a liquid drug via the inlet port or to contract and expel the liquid drug from the elastic sleeve 210.

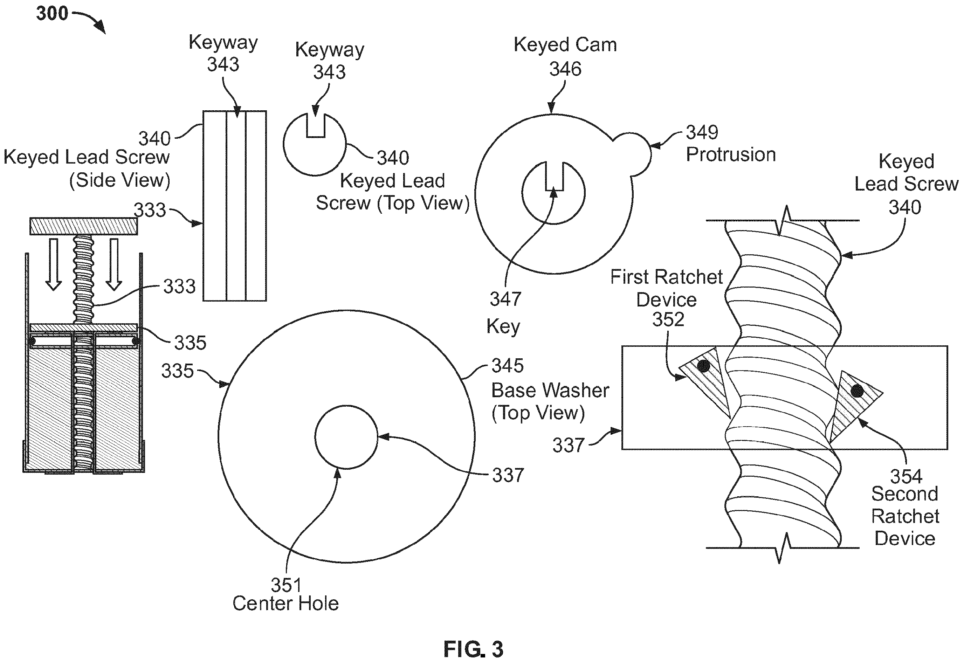

FIG. 3 illustrates a number of features related to an exemplary locking feature according to the disclosed subject matter.

In FIG. 3, a reservoir 300 is shown and exemplary locking features are shown in more detail. For example, arrow 333 points to a side view of the lead screw 340 which shows a keyway 343 extending down the length of the lead screw 340. To the upper right is a top view of a keyed cam 346 that has a key 347 that is configured to slide down the keyway 343 of the lead screw 340 and, optionally, a protrusion 349. The keyed cam 346 is described further with reference to a later example.

Arrow 335 points to a top view of base washer 345. In this example, base washer 345 is circular with a center hole 351 through which may pass the lead screw 340. However, the base washer 345 may also be elliptical with a hole that permits the lead screw 340 to pass. The base washer 345 may further include a first ratchet device 352 and a second ratchet device 354. The first ratchet device 352 and the second ratchet device 354 may be built into, molded into, 3D printed, or otherwise fabricated, while the base washer 345 is fabricated.

The first ratchet device 352 and the second ratchet device 354 may be one-way ratchet features that may be engaged only when the reservoir 300 is filled with the liquid drug and may be configured to permit the base washer 345 to move toward the reservoir cap (shown in previous examples) when expelling the liquid drug from the filled reservoir 300.

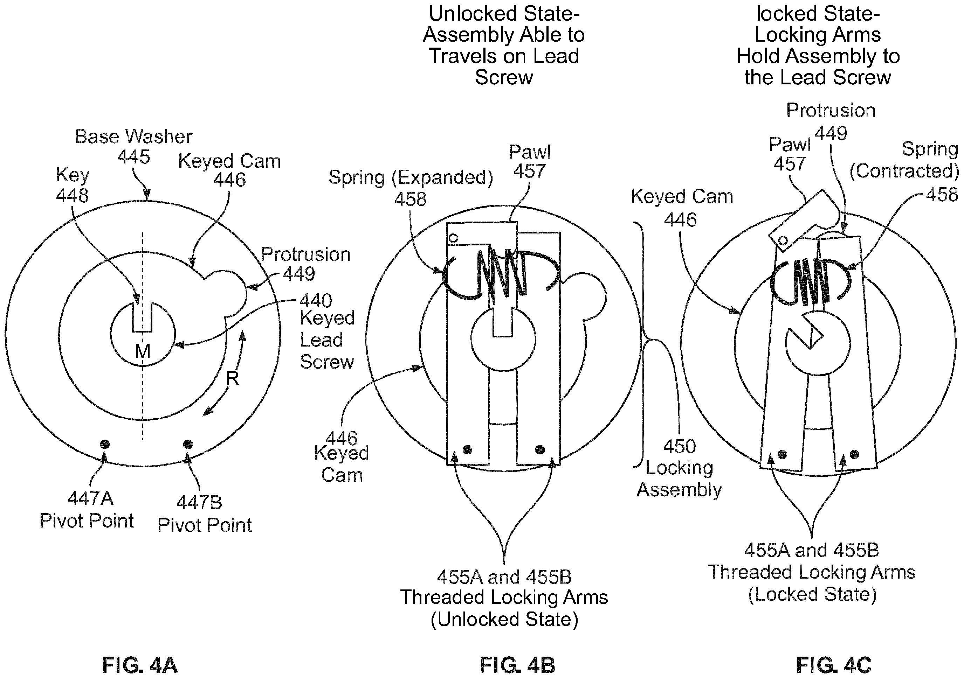

FIG. 4A illustrates an exemplary locking feature for a drug delivery device reservoir according to the disclosed subject matter.

The base washer 445 is similar to the base washers of the examples of FIGs. 1A-3. In the example of FIG. 4A, the base washer 445 has a center opening through which passes a keyed lead screw 440. Positioned over the base washer 445 is a keyed cam 446. The keyed cam 446 is also circular and has a center opening into which extends a key 448 along the axis M. Extending from the perimeter of the keyed cam 446 is a protrusion 449. The protrusion 449 may be approximately at a 45 degree angle with the axis M. The base washer 445 may also have pivot points, pivot point 447A and pivot point 448A. The keyed cam 446 is operable to rotate with the keyed lead screw 440. For example, as the keyed lead screw 440 rotates in a direction, the keyed cam 446 may be operable to rotate in the same direction. The keyed cam 446 may rotate as shown by Arrow R.

FIG. 4B illustrates further details of the exemplary locking feature illustrated in FIG. 4A. FIG. 4B illustrates an unlocked state of the locking assembly 450. In FIG. 4B, a locking assembly 450 is shown positioned over the keyed cam 446 and about the keyed lead screw 440. The locking assembly 450 includes threaded locking arms 455A, 455B, a spring 458, and a pawl 457. The threaded locking arms 455A and 455B include edges that, when in contact with the keyed lead screw 440, prevent the base washer 445 from moving upwards or downwards on the keyed lead screw 440. The threaded locking arms 455A and 455B may couple respectively to the pivot points 447A and 447B.

In the unlocked state of FIG. 4B, the pawl 457 is positioned between the respective threaded locking arms 455A and 455B which prevents the threaded locking arms 455A and 455B from contacting the keyed lead screw 440. In the unlocked state, the base washer 445 is operable to be driven downward by the keyed lead screw 440. The spring 458 may be expanded due to the force applied by the pawl 457. For example, the pawl 457 may be spring-loaded with a spring having sufficient force to position the pawl 457 between the threaded locking arms 455A and 455B by overcoming the force of the spring 458. Of course, other mechanisms besides a spring may be used to move the pawl 457. In the unlocked state of FIG. 4B, for example, the locking assembly 450 as well as the base washer 445 and keyed cam 446 may travel up and down the keyed lead screw 440 as the reservoir is being filled or as liquid drug is being expelled. In an alternative example, the locking assembly 450 may travel up and down the keyed lead screw 440.

While in the unlocked position of FIG. 4B, the wearable drug delivery device is operable to expel any liquid drug from the reservoir for delivery to a user.

FIG. 4C illustrates further details of the exemplary locking feature illustrated in FIGs. 4A and 4B. As the keyed lead screw 440 rotates, the keyed cam 446 also rotates and the protrusion 449 circles around and engages the pawl 457. The keyed lead screw 440 has sufficient torque and the shape of the protrusion 449 that when the protrusion 449 engages the pawl 457, the pawl 457 pushed out from between the threaded locking arms 455A and 455B. As a result of the pawl 457 being out from between the threaded locking arms 455A and 455B, the threaded locking arms 455A and 455B clamp against, or insert ratchets in threads of, the keyed lead screw 440 and prevents additional movement of the base washer 445 up and down the lead screw 440. In the locked state, the base washer 445 may be held in place by the locking assembly 450.

Mechanisms and techniques for maintaining position of the plunger and base washer other than a combination of the keyed cam 446, pawl 457 or threaded locking arms 455A and 455B are envisioned and other examples are described in U.S. Provisional Application No.: 63/375,986, filed on September 16, 2022, the entire content of which is incorporated by reference herein.

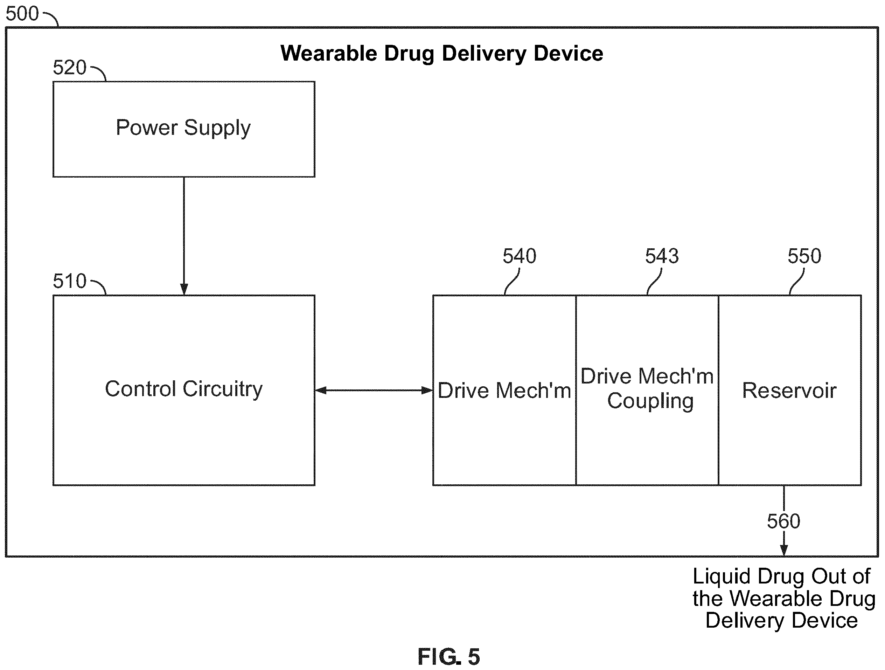

FIG. 5 illustrates a block diagram of an exemplary wearable drug delivery device suitable for using the reservoir illustrated and described herein.

The wearable drug delivery device 500 may include control circuitry 510, a power supply 520, a drive mechanism 540, a drive mechanism coupling 543, and a reservoir 550. The wearable drug delivery device 500 may be a wearable device that is worn on the body of the user. The wearable drug delivery device 500 may be directly coupled to a user (i.e., directly attached to a body part and/or skin of the user via an adhesive, or the like). In an example, a surface of the wearable drug delivery device 500 may include an adhesive to facilitate attachment to the skin of a user.

The reservoir 550, such as those described herein, may store a liquid drug. In addition, while the device 500 is described with reference to delivery of insulin and the use of an AID algorithm, the device 500 may be operable to implement a drug delivery regimen via a medication delivery algorithm using a number of different liquid or therapeutic drugs. Examples of a liquid drug may be or include any drug in liquid form capable of being administered by a drug delivery device via a subcutaneous cannula, including, for example, insulin, glucagon-like peptide-1 (GLP-1), pramlintide, glucagon, co-formulations of two or more of GLP-1, pramlintide, and insulin; as well as pain relief drugs, such as opioids or narcotics (e.g., morphine, or the like), methadone, arthritis drugs, hormones, such as estrogen and testosterone, blood pressure medicines, chemotherapy drugs, fertility drugs, or the like.

The elastic sleeve of this disclosure may be made of an elastomeric material having an elongation at break of at least 300% more preferably at least 500%.

The drive mechanism 540 may be responsive to signals from the control circuitry and may include a number of mechanical elements such as gears, gear trains and the like. The drive mechanism 540 may be operable to drive a lead screw such as those described in earlier examples.

A drive mechanism coupling 543 may couple the drive mechanism 540 to the lead screw and to the reservoir 550. The drive mechanism coupling 543 may translate linear motion into rotary motion, or the like. The drive mechanism coupling 543 may have the structural elements that enable a precise amount of a liquid drug stored in the reservoir 550 to be expelled from the reservoir 550 via a fluid pathway 560 from the wearable drug delivery device 500.

The power supply 520 may be an electrical power source, such as a battery, a super capacitor, an energy harvesting circuit or the like. The power supply 520 may be configured to last several hours, several days or the like. The power supply 520 may be replaceable and/or rechargeable via wired connections or wireless connections.

The wearable drug delivery device 500 may include control circuitry 510 that may be implemented in hardware, software, or any combination thereof. The control circuitry 510 may, for example, be a microprocessor, a logic circuit, a field programmable gate array (FPGA), an application specific integrated circuit (ASIC) or a microprocessor coupled to a memory. The control circuitry 510 may be operable to perform a number of functions, such as executing a control application stored in the memory (not shown) to enable the functions described herein, such as rotation of the lead screw within the reservoir 550.

A type of medication delivery algorithm (MDA) may include an "artificial pancreas" algorithm-based system, or more generally, an artificial pancreas (AP) application. For ease of discussion, the computer programs and computer applications that implement the medication delivery algorithms or applications may be referred to herein as an "AP application." An AP application may be configured to provide automatic delivery of insulin based on an analyte sensor input, such as signals received from an analyte sensor, such as a continuous blood glucose monitor, ketone sensor, or the like. The signals from the analyte sensor may contain blood glucose measurement values, timestamps, or the like.

Although the housing of the reservoir as well as the plunger mentioned in this disclosure may both have a cylindrical cross-sectional shape, it should be understood that the housing and the plunger of the present disclosure may also have a non-cylindrical cross-sectional shape. This has the advantage that the plunger cannot rotate within the housing when the lead screw rotates to advance the plunger within the housing. A cylindrical shape is also possible since the threading of the lead screw mainly exerts axial forces on the plunger or the base washer, respectively, if the pitch of the threading is not too large, such that the plunger does not rotate when the lead screw rotates.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first" and "second" are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

Although the present invention is defined in the enclosed claims, it should be understood that the present invention can alternatively be defined in accordance with the following embodiments:
1. A drug delivery system comprising a reservoir comprising a housing (120), wherein the housing (120) has a first end (123) and a second end (125); a plunger (130; 230) which is moveable within the housing (120); characterized in that the reservoir further comprises an elastic sleeve (110), wherein one end portion of the elastic sleeve (110) is coupled to the plunger, and wherein another end portion of the elastic sleeve (110) is coupled to a reservoir cap (125; 225).
2. A drug delivery system according to embodiment 1, wherein the reservoir has a volume for storing a liquid drug which is enclosed by the plunger (130; 230), an inner surface of the housing (120), the reservoir cap (125) and the elastic sleeve (110).
3. A drug delivery system according to embodiment 1 or 2, further comprising a lead screw (140; 240; 340) passing through the plunger (130; 230) and through the elastic sleeve (110), in particular through the center of the plunger (130; 230) and through the center of the elastic sleeve (110).
4. A drug delivery system according to one of the preceding embodiments, wherein the elastic sleeve (110) provides a leakproof connection between the plunger (130; 230) and the reservoir cap (125; 225).
5. A drug delivery system according to one of the preceding embodiments, wherein the sleeve (110) is connected to the plunger (130; 230) at a side of the plunger which is facing the first end (123) of the housing.
6. A drug delivery system according to one of the preceding embodiments, wherein an axially extending portion of the elastic sleeve (110) which is arranged between the plunger (130; 230) and the reservoir cap (125; 225) has an unextended length when the plunger (130; 230) contacts the reservoir cap (125) and an extended length when the plunger is moved away from the reservoir cap (125) towards the first end of the housing.
7. A drug delivery system according to embodiment 6, wherein the plunger has an axial extension such that the unextended length of the axially extending portion of the elastic sleeve (110) is arranged within the center of the plunger, preferably an axial extension in the range of 1.5 to 5 mm, most preferably an axial extension in the range of 2 to 4 mm.
8. A drug delivery system according to one of the preceding embodiments, wherein the elastic sleeve is operable to expand as the plunger is moved away from the reservoir cap; and the elastic sleeve is operable to contract when the plunger is moved toward the reservoir cap.
9. A drug delivery system according to one of the preceding embodiments, further comprising a drive mechanism (540) for rotating the lead screw, wherein rotation of the lead screw results in an axial movement of the plunger towards the second end of the housing, and optionally a control circuitry (510), a power supply (520), and/or a drive mechanism coupling (543).
10. A drug delivery system according to one of the preceding embodiments, wherein a rotation of the plunger is prevented, in particular by a non-circular cross-sectional shape of the plunger and a corresponding cross-sectional shape of the housing.
11. A drug delivery system according to one of the preceding embodiments, wherein a base washer (145; 245; 345) is connected to the plunger or integrally formed to the plunger.
12. A drug delivery system according to embodiment 11, wherein the base washer has an internal threading which engages the threading of the lead screw, or a base washer lock (247) which permits the plunger (230) and base washer (245) to rise freely in the direction away from the reservoir cap (225) along the length of the lead screw (240) when the reservoir (220) is being filled with a liquid drug (288), and which enables precise control of the amount of liquid drug (288) being dispensed by engaging the lead screw (240) when the lead screw (240) drives the base washer (247) downward toward the reservoir cap (225).
13. A drug delivery system according to one of the preceding embodiments, wherein the base washer has threaded locking arms (455A, 455B) or first and second ratchet devices (352, 354).
14. A drug delivery system according to embodiment 13, further comprising a keyed cam (346) which is coupled to the plunger and/or to the base washer, wherein the keyed cam has a key (347) engaging a keyway (343) in the lead screw such that the keyed cam rotates together with the lead screw and is axially movable along the lead screw, wherein a pawl (457) initially keeps the threaded locking arms (455A, 455B) disengaged from the lead screw, and wherein the keyed cam (346) comprises a protrusion (449) which acts to disengage the pawl (457) upon rotation of the keyed cam such that the threaded locking arms (455A, 455B) engage the lead screw, preferably by pulling the threaded locking arms together by the force of a spring (458).
15. A drug delivery system as defined in the preceding embodiments, wherein the reservoir cap (125) is fixedly connected to the second end (125) of the housing (120), in particular by bonding, welding, friction, snaps or threads, or is an integral part of the housing (120) at its second end (125).
16. A drug delivery system as defined in the preceding embodiments, wherein the reservoir cap (125) seals the second end (125) of the housing.
17. A drug delivery system as defined in the preceding embodiments, wherein the housing (120) or the reservoir cap (125) comprises at least one port, in particular an inlet port (161) and an outlet port (163).
18. A drug delivery system as defined in the preceding embodiments, wherein the plunger further comprises a fluid stop perimeter having a leakproof sealing component, wherein the leakproof sealing component engages an internal wall of the cylindrical housing.
19. A drug delivery system, comprising a reservoir having a rigid housing wherein the rigid housing has a first end and a second end; a plunger configured to fit within the rigid housing; a leadscrew coupled to the plunger; and an elastic sleeve surrounding the leadscrew and coupled to a surface of the plunger and to a surface of the second end of the rigid housing, wherein the elastic sleeve is leakproof.
20. The drug delivery system of embodiment 19, wherein the plunger is further configured traverse within the rigid housing along a length of the lead screw.
21. The drug delivery system of embodiment 19 or 20, wherein the elastic sleeve is further configured to traverse with the plunger, expand and contract based on the direction that the plunger traverses, and maintain a boundary between a liquid drug in the reservoir and the leadscrew.
22. The drug delivery system of one of embodiments 19 to 20, wherein the lead screw is coupled to a center of the plunger.
23. The drug delivery system of one of embodiments 19 to 22, further comprising: an inlet port through the second end of the rigid housing and through which a liquid drug fills a void between the plunger and the second end.

## Claims

1. A drug delivery system comprising:
a reservoir having a housing,
a plunger configured to fit within the housing,
an elastic sleeve within the housing and coupled to a surface of the plunger, wherein the elastic sleeve is leakproof,
a lead screw passing through a center of the elastic sleeve and a center of the plunger, and
a reservoir cap coupled to the housing.

2. The drug delivery system of claim 1, wherein the plunger further comprises: a fluid stop perimeter having a leakproof sealing component, wherein the leakproof sealing component engages an internal wall of the cylindrical housing.

3. The drug delivery system of claim 1 or 2, wherein rotation of the lead screw is operable to move the plunger within the cylindrical housing.

4. The drug delivery system of one of the preceding claims, wherein the reservoir cap is stationary.

5. The drug delivery system of one of the preceding claims, wherein the reservoir cap includes a sealed port coupled through the reservoir cap that enables a void between the plunger and the reservoir cap to be filled with a liquid drug.

6. The drug delivery system of one of the preceding claims, wherein the lead screw has a length greater than the cylindrical housing.

7. The drug delivery system of one of the preceding claims, wherein plunger and the cylindrical housing are of the same shape, and the plunger

8. The drug delivery system of one of the preceding claims, wherein the cylindrical housing is a rigid structure.

9. The drug delivery system of one of the preceding claims, further comprising: a threaded nut having threads that engage threads of the lead screw, wherein the lead screw is operable to advance the threaded nut enabling the elastic sleeve to expand to fill the elastic sleeve with a liquid drug via the port or enabling the elastic sleeve to contract to expel the liquid drug from the elastic sleeve.

10. The drug delivery system of one of the preceding claims, wherein the reservoir cap further comprises: an exit port operable to enable the liquid drug to be expelled from the elastic sleeve in response movement of the plunger.

11. The drug delivery system of one of the preceding claims, wherein movement of the plunger is in response to rotation of the lead screw.

12. The drug delivery system of one of the preceding claims, wherein the elastic member is positioned between the base washer and the plunger, and extends radially through a center opening of the plunger and is sealed to a surface of the plunger facing the base washer.

13. The drug delivery system of one of the preceding claims, wherein: the elastic sleeve is operable to expand as the plunger is moved away from the reservoir cap; and the elastic sleeve is operable to contract when the plunger is moved toward the reservoir cap.

14. The drug delivery system of one of the preceding claims, wherein: the reservoir has an initial fill position when the plunger is located at a location nearest to the reservoir cap within the cylindrical housing; and the reservoir has a filled position when the plunger is located at a location farthest away from the reservoir cap within the cylindrical housing.

15. The drug delivery system of one of the preceding claims, wherein: the reservoir cap is coupled to an end of the cylindrical housing by bonding, welding, friction, snaps or threads.
